# NEW EUROPEAN PATENT SPECIFICATION

(11) **EP 1 589 958 B2**
(45) Date of publication and mention of the opposition decision: **11.04.2012**
(45) Mention of the grant of the patent: 27.05.2009
(21) Application number: 04702017.7
(22) Date of filing: 14.01.2004
(51) Int. Cl.: A61K 31/135, A61P 13/10, A61K 31/216, A61K 31/4025, A61K 31/439

(54) **REDUCED DOSE OF TOLTERODINE FOR TREATING URINARY DISORDERS**
HERABGESETZTE DOSIS VON TOLTERODIN ZUR BEHANDLUNG VON HARNWEGSERKRANKUNGEN
UTILISATION D'UNE DOSE REDUITE DE TOLTERODINE POUR TRAITER DES TROUBLES URINAIRES

(30) Priority: 22.01.2003 US 441690 P
(43) Date of publication of application: 02.11.2005
(73) Proprietor: Pfizer Health AB, 112 87 Stockholm (SE)
(72) Inventor: KORBERLY, Barbara, H.,Pfizer Consumer Healthcare, Morris Plains, NJ 07950 (US); DANEHOWER, Susan, M.,Pfizer Consumer Healthcare, Morris Plains, NJ 07950 (US)
(74) Representative: Hayles, James Richard
(86) International application number: PCT/IB2004/000169
(87) International publication number: WO 2004/064821

(56) References cited:
- WO-A-00/12069
- WO-A-01/34139
- DATABASE MEDLINE [Online] US NATIONAL LIBRARY OF MEDICINE (NLM), BETHESDA, MD, US; JONAS U ET AL: "Efficacy and safety of two doses of tolterodine versus placebo in patients with detrusor overactivity and symptoms of frequency, urge incontinence , and urgency: urodynamic evaluation. The International Study Group." XP002279460 retrieved from STN Database accession no. 97290150 & WORLD JOURNAL OF UROLOGY. JOURNAL CODE: 8307716. ISSN: 0724-4983., vol. 15, no. 2, 1997, pages 144-151,
- DATABASE MEDLINE [Online] US NATIONAL LIBRARY OF MEDICINE (NLM), BETHESDA, MD, US; APPELL R A: "Clinical efficacy and safety of tolterodine in the treatment of overactive bladder : a pooled analysis." XP002279461 retrieved from STN Database accession no. 1998088166 & UROLOGY, 50; DISCUSSION 97-9. JOURNAL CODE: 0366151. ISSN: 0090-4295., vol. 50, December 1997 (1997-12), pages 90-96,
- DATABASE MEDLINE [Online] US NATIONAL LIBRARY OF MEDICINE (NLM), BETHESDA, MD, US; RENTZHOG L ET AL: "Efficacy and safety of tolterodine in patients with detrusor instability: a dose-ranging study." XP002279462 retrieved from STN Database accession no. 1998128604 & BRITISH JOURNAL OF UROLOGY. JOURNAL CODE: 15740090R. ISSN: 0007-1331., vol. 81, no. 1, January 1998 (1998-01), pages 42-48,
- DATABASE MEDLINE [Online] US NATIONAL LIBRARY OF MEDICINE (NLM), BETHESDA, MD, US; VAN KERREBROECK P E ET AL: "Dose-ranging study of tolterodine in patients with detrusor hyperreflexia." XP002279463 retrieved from STN Database accession no. 1998446908 & NEUROUROLOGY AND URODYNAMICS. JOURNAL CODE: 8303326. ISSN: 0733-2467., vol. 17, no. 5, 1998, pages 499-512,
- DATABASE MEDLINE [Online] US NATIONAL LIBRARY OF MEDICINE (NLM), BETHESDA, MD, US; SUSSMAN DAVID ET AL: "Treatment of overactive bladder with once-daily extended-release tolterodine or oxybutynin: the antimuscarinic clinical effectiveness trial (ACET)." XP002279464 retrieved from STN Database accession no. 2002446267 & CURRENT MEDICAL RESEARCH AND OPINION. JOURNAL CODE: 0351014. ISSN: 0300-7995., vol. 18, no. 4, 2002, pages 177-184,
- DATABASE MEDLINE [Online] US NATIONAL LIBRARY OF MEDICINE (NLM), BETHESDA, MD, US; OLSSON B ET AL: "Multiple dose pharmacokinetics of a new once daily extended release tolterodine formulation versus immediate release tolterodine." XP002279465 retrieved from STN Database accession no. 2001504832 & CLINICAL PHARMACOKINETICS. JOURNAL CODE: 7606849. ISSN: 0312-5963., vol. 40, no. 3, 2001, pages 227-235,
- DATABASE MEDLINE [Online] US NATIONAL LIBRARY OF MEDICINE (NLM), BETHESDA, MD, US; VAN KERREBROECK P ET AL: "Tolterodine once-daily: superior efficacy and tolerability in the treatment of the overactive bladder ." XP002279466 retrieved from STN Database accession no. 2001227609 & UROLOGY. JOURNAL CODE: 0366151. ISSN: 1527-9995., vol. 57, no. 3, March 2001 (2001-03), pages 414-421,
- DATABASE MEDLINE [Online] US NATIONAL LIBRARY OF MEDICINE (NLM), BETHESDA, MD, US; NILVEBRANT L ET AL: "Antimuscarinic potency and bladder selectivity of PNU-200577, a major metabolite of tolterodine." XP002279467 retrieved from STN Database accession no. 1998015289 & PHARMACOLOGY & TOXICOLOGY. JOURNAL CODE: 8702180. ISSN: 0901-9928., vol. 81, no. 4, October 1997 (1997-10), pages 169-172,
- DATABASE MEDLINE [Online] US NATIONAL LIBRARY OF MEDICINE (NLM), BETHESDA, MD, US; YAMAUCHI K ET AL: "Clinical effects of oxybutynin hydrochloride (Pollakis)--especially in the treatment of pollakisuria, urgency and urinary incontinence ." XP002279468 retrieved from STN Database accession no. 91165719 & HINYOKIKA KIYO. ACTA UROLOGICA JAPONICA. JOURNAL CODE: 0421145. ISSN: 0018-1994., vol. 36, no. 12, December 1990 (1990-12), pages 1485-1490,
- DATABASE MEDLINE [Online] US NATIONAL LIBRARY OF MEDICINE (NLM), BETHESDA, MD, US; YOKOYAMA E ET AL: "Clinical effect of oxybutynin hydrochloride ( 1 mg /tablet)." XP002279469 retrieved from STN Database accession no. 91051049 & HINYOKIKA KIYO. ACTA UROLOGICA JAPONICA. JOURNAL CODE: 0421145. ISSN: 0018-1994., vol. 36, no. 7, July 1990 (1990-07), pages 869-876,
- DATABASE MEDLINE [Online] US NATIONAL LIBRARY OF MEDICINE (NLM), BETHESDA, MD, US; TOMA H ET AL: "Clinical effects of oxybutynin hydrochloride on neurogenic bladder ." XP002279470 retrieved from STN Database accession no. 87022593 & HINYOKIKA KIYO. ACTA UROLOGICA JAPONICA. JOURNAL CODE: 0421145. ISSN: 0018-1994., vol. 32, no. 6, June 1986 (1986-06), pages 907-911,
- 'ROTE LISTE 1999', 1999 article 'Detrusitol 1mg/ -2mg'
- LARSSON G. ET AL.: 'Tolterodine in the treatment of overactive bladder: Analysis of the pooled phase II efficacy and safety data' UROLOGY vol. 53, no. 5, 1999, pages 990 - 998
- JACQUETIN B. ET AL.: 'Tolterodine reduces the number of urge incontinence episodes in patients with an overactive bladder' EUROPEAN JOURNAL OF OBSTETRICS & GYNECOLOGY AND REPRODUCTIVE BIOLOGY vol. 98, 2001, pages 97 - 102
- STAHL M. M. S. ET AL.: 'Urodynamic and other effects of tolterodine: A novel antimuscarinic drug for the treatment of detrusor overactivity' NEUROUROLOGY AND URODYNAMICS vol. 14, 1995, pages 647 - 655
- BRYNNE N. ET AL.: 'Pharmacokinetics and pharmacodynamics of tolterodine in man: a new drug for the treatment of urinary bladder overactivity' INTERNATIONAL JOURNAL OF CLINICAL PHARMACOLOGY AND THERAPEUTICS vol. 35, no. 7, 1997, pages 287 - 295

## Description

### Field of invention

The present invention relates to oral methods for treating unstable or overactive urinary bladder in a mammal while minimizing adverse events and side-effects such as the occurrence of dry mouth, dyspepsia and reduced stream of tears. These methods comprise orally administering to a mammal a pharmaceutically effective dose of tolterodine or salt thereof on an as needed, prn basis, whereby a symptomatic relief of urgency and/or frequency is achieved.

### Background of the invention

A substantial part (5-10%) of the adult population suffers from urinary incontinence, and the prevalence, particularly of so-called urge incontinence, increases with age. The symptoms of an unstable or overactive bladder comprise urge incontinence, urgency and urinary frequency. It is assumed that unstable or overactive bladder is caused by uncontrolled contractions of the bundles of smooth muscle fibers forming the muscular coat of the urinary bladder (the detrusor muscle) during the filling phase of the bladder. These contractions are mainly controlled by cholinergic muscarinic receptors, and the pharmacological treatment of unstable or overactive bladder has been based on muscarinic receptor antagonists.

The reason why the bladder muscle contracts inappropriately is unclear in many cases. For some people it may be due to a problem with nerve signals that run from the brain to the bladder. Surgery or childbearing sometimes causes minor nerve damage. This muscle squeezes or contracts more often than normal and at inappropriate times. Instead of staying at rest as urine fills the bladder, the detrusor contracts while the bladder is filling with urine. This causes a person to feel a sudden and sometimes overwhelming urge to urinate even when the bladder is not filled.

Overactive urinary bladder encompasses a variety of urinary disorders including overactive detrusor (detrusor instability, detrusor hyperreflexia) and sensory urgency and the symptoms of detrusor overactivity, e.g. urge incontinence, urgency and urinary frequency and LUTS (Lower urinary Tract Symptoms including obstructive urinary symptoms such as slow urination, dribbling at the end of urination, inability to urinate and/or the need to strain to urinate at an acceptable rate or irritate symptoms such as frequency and/or urgency). Also other conditions are included, which give rise to urinary frequency, urgency and/or urge incontinence. Overactive bladder disorders also include nocturia and mixed incontinence. While overactive bladder is often associated with detrusor muscle instability, disorders of bladder function may also be due to neuropathy of the central nervous system (detrusor hyperreflexia) including spinal cord and brain lesions, such as multiple sclerosis and stroke. Overactive bladder symptoms may also result from, for example, male bladder outlet obstruction (usually due to prostatic hypertrophy), interstitial cystitis, local edema and irritation due to focal bladder cancer, radiation cystitis due to radiotherapy to the pelvis, and cystitis.

A specific urinary disorder, which can be treated by the claimed method, is a dry overactive bladder, which includes frequency, urgency and nocturia.

Antimuscarinic compounds have been developed for the treatment of urinary disorders such as unstable or overactive bladder. The drug of choice has earlier been oxybutynin (marketed as, for example, Ditropan®). Typically, patients are given 5-15 mg per day for a sustained release formulation, or 5-30 mg per day of an immediate release formulation. Recently, however, an improved muscarinic receptor antagonist, tolterodine, (R)-N,N-diisopropyl-3-(2-hydroxy-5-methylphenyl)-3-phenylpropanamine, has been marketed for the treatment of unstable or overactive bladder with symptoms including urge incontinence, urinary urgency and urinary frequency. Both tolterodine and its major active metabolite, the 5-hydroxymethyl derivative of tolterodine, which significantly contributes to the therapeutic effect, have considerably less side-effects than oxybutynin, especially regarding the propensity to clause dry mouth. While tolterodine is equipotent with oxybutynin in the bladder, its affinity for muscarinic receptors of the salivary grand is eight times lower than that of oxybutynin; see, for example, Nilvebrant L., et al.; European Journal of Pharmacology, 327 (1997) 195-207. The selective effect of tolterodine in humans is described in Stahl, M. M. S., et al., Neurourology and Urodynamies 14 (1995) 647-65, and Brynne, N., International Journal of Clinical Pharmacology and Therapeutics, Vol. 35, No. 7 (1995) 287-295. Tolterodine is presently being sold in a number of different countries for treatment of urinary incontinence under the name Detrol®, marketed by Pharmacia (now part of Pfizer).

As mentioned above, the chemical name of tofterodine is (R)-N,N-diisopropyl-3-(2-hydroxy-5-methylphenyl)-3-phenylpropanamine. Specifically included is tolterodine L- tartrate

Tolterodine, its corresponding (S)-enantiomer and racemate and the preparation thereof are described in e.g. US-A-5,382,600 (WO89/06644). . The (S)-enantiomer, its non-cholinergic spasmolytic activity and use in the treatment of urinary and gastrointestinal disorders are described in WO 98/03067.

The currently marketed administration forms of tolterodine are filmcoated tablet for immediate release and capsules or filmcoated tablets for controlled release. The immediate release tablets contain 1 mg, or 2 mg of tolterodine L-tartrate for immediate release in the gastrointestinal tract. The capsules or filmcoated tablets for oral controlled release formulation for once-daily administration have a dosage of tolterodine or related compound of 2 mg or 4 mg or 6 mg. The recommended dosage is usually 2 mg twice a day for chronic use.

### Description of the Invention

In the present invention it is unexpectedly found that the occurrence of dry mouth, dyspepsia and reduced stream of tears that can be associated with the high dosage of tolterodine can be minimized by administering the tolterodine or a pharmaceutically acceptable salt thereof, on an as needed, prn basis. In other words, it is found that the administration of tolterodine or a pharmaceutically acceptable salt thereof, on an as needed, prn basis causes less side-effects but achieves a symptomatic relief of urgency and/or frequency.

Consumers constantly require alternative methods of administration, especially when the need for medicament treatment is urgent and/or when the patient has an active lifestyle. Thus, the administration method of this invention will be especially beneficial in treating these above-mentioned consumers. Furthermore, from a patient lifestyle standpoint the methods of the present invention would also be more convenient than the usual earlier recommended methods of administration, requiring chronic dosing of, for example, 2x 2mg tolterodine daily permanently.

For these and other purposes, it is an object of the present invention to provide a method of administration, which method brings symptomatic relief from symptoms arising from said urinary disorder such as e.g. urinary urgency and/or frequency.

The present invention provides the use of tolterodine, or a pharmaceutically acceptable salt thereof, for the manufacture of a medicament for acute oral administration on an as needed, prn basis, to a human with unstable or overactive urinary bladder, whereby a symptomatic relief of urgency and/or frequency is achieved, wherein two pharmaceutically effective doses of 1 mg tolterodine, or a pharmaceutically acceptable salt thereof, are administered daily at an interval of 8-12 hours, as an immediate release tablet or capsule.

The invention will now be described by way of example. Methods routinely applied by the skilled person are used, in addition to those described in detail in the Examples below.

### Example 1

The objective of a study is to evaluate the efficacy and safety of tolterodine tartrate (Detrol®) tablets 2 mg daily versus placebo, in the treatment of urinary urgency and frequency in women, in a randomized, double-blind, placebo-controlled study. The patients received 1 mg tolterodine b.i.d. or placebo, and the dosage regime was one tablet orally, every 8-12 hours, not to exceed 2 tablets daily. The treatment duration was 10 days, after a 5 day pretreatment baseline phase. 1315 women were included in the study, and were randomized to either the tolterodine or placebo group. All of the subjects were eligible for intent-to-treat (ITT) and safety analysis. 1077 of the randomized subjects were included in the per-protocol analysis.

Females with symptoms of urinary urgency, defined as having to stop the current activity and go immediately to the bathroom at least once a day (severe) or able to finish a task but go right to the bathroom at least twice a day (moderate), were included. Frequency was defined as >7 micturitions per 24 hours. The mean age was 48 years. About 83% of the subjects were Caucasian. The two treatment groups were comparable with respect to the demographic data.

Women were asked to complete a diary recording the time of awakening; number and severity of urgency of each micturition, and the number of episodes of incontinence throughout the day as well as, time when tablets taken. The women were directed to begin using the study drug after 5 days (baseline, pretreatment period): 1 tablet every 8 - 12 hours not to exceed 2 tables per day.

Thus, this study was designed to evaluate the efficacy and safety of tolterodine 1 mg twice daily, every 8-12 hours, compared to placebo, in women with urinary urgency and frequency. The primary efficacy endpoints were:
1. Subject perception of improvement in symptoms of urgency after five days of treatment, vs. baseline period, using a three-point categorical scale (Chi Square analysis):
2. Mean of daily average severity of urgency for urgent episodes (urgency rating score of at least 1) for the 5-day baseline period vs. first five days of treatment.

A five-point categorical scale was used to assess the severity of urgency experienced at each micturition, ranging from 0 (no discomfort) to 4 (very severe).

The secondary efficacy endpoints were:
1. Average number of urgency episodes per day per subject for the 5-day baseline period vs. first five days of treatment.
2. Average urgency score (severity of urgency) per subject for micturition episodes accompanied by urgency (a rating score of at least 1) for the 5-day baseline period vs. first day of treatment.
3. Average number of micturitions per day per subject for the 5-day baseline period vs. first five days of treatment.

Subject perception of improvement in symptoms or urgency after ten days of treatment, vs. baseline period, using a three-point categorical scale (Chi Square analysis).

The sudden urge to urinate accompanied by frequency of urination is a problem for many adults. The symptoms of urgency are most problematic. Therefore, the primary variables examined the subjective perception of improvement in urgency as well as the severity of urgency in subjects treated with tolterodine 1 mg b.i.d. In addition, the frequency of urination was examined as this is a standard assessment tool commonly used to assess the efficacy of drugs for the treatment of overactive bladder syndrome.

Two populations were analyzed for efficacy: the intent-to-treat (ITT) and the per-protocol populations. The former comprised all individuals randomized to treatment. The latter consisted of those individuals who complied with the protocol with regard to eligibility, visit schedule, diary completion and treatment schedule. All randomized subjects who took study medication were included in the safety analyses. Incidence of adverse events was tabulated for all randomized subjects.

The data obtained from this study were assessed by descriptive statistics, as well as by appropriate non-parametric and parametric (ANOVA) comparisons of the two treatment groups for efficacy and safety. More specifically, subject perception of improvement in symptoms of urgency were analyzed using Cochran-Mantel-Haenszel (CMH) test, controlling for center effect. Unless otherwise stated, change from baseline variables were analyzed via an ANOVA model including treatment and center effects. Treatment-by-center effects were assessed by adding treatment-by-center term to the original model. If treatment-by-center interaction was detected at 0.05 level, the interaction would be assessed. If the source of the interaction could be traced to one or two aberrant centers, then an additional analysis would be performed excluding those centers.

### RESULTS:

The tolterodine group had significantly higher baseline severity compared to the placebo group for three efficacy variables: mean of daily average severity of urgency at micturition score (1.99 vs 1.94, p=0.041), mean of daily average severity for urgent episodes score (2.10 vs 2.06, p=0.046), average daily number of pads used (0.62 vs 0.49, p=0.05). To account for these imbalances, it was decided post hoc to include the baseline effect in the analysis model.

### The efficacy results:

Subject perception of improvement in symptoms of urgency on Day 5 was one of the two primary efficacy variables. The tolterodine group had a statistically significantly higher percentage of subjects reporting symptom improvement on Day 5 compared to placebo. Similar results were observed on Day 10.
Severity of urgency for all micturitions: The tolterodine group had a statistically significantly greater decrease in severity of urgency, compared to placebo, for all analyzed time points, including day 1.
Severity of urgency for urgent episodes: The tolterodine group had a statistically significantly greater decrease in severity of urgency for urgent episodes, compared to placebo, for all analyzed time points, including day 1. For the primary time point of interest (days 1-5), the improvement from baseline was 0.27 for the tolterodine group and 0.19 for the placebo group.
Number of micturitions/day: The tolterodine group had a statistically significantly greater decrease in the number of micturitions, compared to placebo, for all analyzed time points. For example for days 1-5, a reduction from baseline of 1.59 was achieved in the tolterodine-treated group, whereas only a reduction from baseline of 1.26 was achieved in the placebo group (N=660 for tolterodine, N=655 for placebo).

Number of urgent episodes/day: The tolterodine group had a statistically significantly greater reduction in the number of urgent episodes, compared to placebo, for all analyzed time points.

To summarise, the tolterodine group had, compared to placebo, statistically significant:
o Greater percentage of subjects experiencing improvement;
o Lower urgency severity scores;
o Fewer number of urgent episodes per day;
o Fewer number of micturitions per day.
Overall, these results demonstrate that tolterodine 1 mg b.i.d daily is effective in treating symptoms of urinary urgency and frequency with acute treatment. Importantly, the perceived sense of urgency decreased significantly after the first day of treatment.

### The safety results:

Seventy-one (10.8%) subjects in the tolterodine group and 70 (10.7%) subjects in the placebo group reported adverse events. Of these, thirty-two (4.8%) subjects in the tolterodine group and 31 (4.7%) subjects in the placebo group had adverse events that were considered related to study medication by the investigators.

Three subjects in the tolterodine group and one subject in the placebo group reported serious AEs. Three subjects in the tolterodine group and four subjects in the placebo group discontinued from the study due to AEs. AEs related to gastrointestinal disorders were the most commonly reported: 37 (5.6%) tolterodine subjects and 32 (4.9%) placebo subjects. AEs related to nervous system disorders were the next most commonly reported: 20 (3.0%) subjects in the tolterodine group and 13 (2.0%) subjects in the placebo group.

### CONCLUSION:

This study demonstrates that tolterodine 1 mg b.i.d. is effective in decreasing the severity of the sensation of urgency and the frequency of micturitions in the population studied. Additionally, significantly more subjects in the tolterodine group, compared to the placebo group, reported improvement in their symptoms. Importantly, improvements were seen by the first day of treatment and continued throughout the treatment period.

This suggests that a woman experiencing urinary urgency and/or frequency can expect to see a beneficial impact of tolterodine treatment with acute use. This is significant as it allows those who experience these symptoms to treat only as needed, when symptoms are or are anticipated to be bothersome (e.g. a restroom is not readily assessable or frequent trips to the restroom are not feasible). The rapid onset of therapeutic benefit allows for treatment to be instituted on an "as needed" (prn) basis, based on a person's desire to control symptoms.

## Claims

1. Use of tolterodine, or a pharmaceutically acceptable salt thereof, for the manufacture of a medicament for acute oral administration on an as needed, prn basis, to a human with unstable or overactive urinary bladder, whereby a symptomatic relief of urgency and/or frequency is achieved, wherein two pharmaceutically effective doses of 1 mg tolterodine, or a pharmaceutically acceptable salt thereof, are administered daily at an interval of 8-12 hours as an immediate release tablet or capsule.

## Patentansprüche

1. Verwendung von Tolterodin oder einem pharmazeutisch verträglichen Salz davon zur Herstellung eines Medikaments zur akuten oralen Verabreichung bei Bedarf, auf prn-Basis, an einen Menschen mit instabiler oder überaktiver Harnblase, wobei eine symptomatische Erleichterung von Drang und/oder Häufigkeit erreicht wird, wobei zwei pharmazeutisch wirksame Dosen von 1 mg Tolterodin oder einem pharmazeutisch verträglichen Salz davon täglich in einem Intervall von 8-12 Stunden als eine Tablette oder Kapsel mit sofortiger Freisetzung verabreicht werden.

## Revendications

1. Utilisation de toltérodine, ou d'un de ses sels pharmaceutiquement acceptables, pour la production d'un médicament pour l'administration orale aiguë, de la manière requise, suivant les besoins, à un être humain présentant une instabilité ou hyperactivité vésicale, ce qui permet d'obtenir un soulagement symptomatique du besoin impérieux et/ou de la fréquence, dans laquelle deux doses pharmaceutiquement efficaces de 1 mg de toltérodine, ou d'un de ses sels pharmaceutiquement acceptables, sont administrées quotidiennement à un intervalle de 8 à 12 heures sous forme de comprimé ou capsule à libération immédiate.
